# EUROPEAN PATENT SPECIFICATION

(11) **EP 0 364 245 B1**
(45) Date of publication and mention of the grant of the patent: **02.03.1994**
(21) Application number: 89310405.9
(22) Date of filing: 11.10.1989
(51) Int. Cl.: A61K 7/22, A61K 7/16

(54) **Dentifrice compositions**
Zahnreinigungsmittel
Compositions dentifrices

(30) Priority: 13.10.1988 GB 8824073; 30.11.1988 GB 8827913; 01.08.1989 GB 8917580
(43) Date of publication of application: 18.04.1990
(60) Divisional application: 93202062.1
(73) Proprietor: BEECHAM GROUP PLC, Brentford, Middlesex TW8 9BD (GB)
(72) Inventor: Alexander, Stephen Edward Beecham Prod. Res.Dep., Weybridge Surrey KT13 0DE (GB); Doel, Geoffrey Royston Beecham Prod. Res. Dep., Weybridge Surrey KT13 0DE (GB); Edwards, Peter John Beecham Prod. Res. Dep., Weybridge Surrey KT13 0DE (GB)
(74) Representative: Connell, Anthony Christopher

(56) References cited:
- EP-A- 0 026 252
- DE-A- 2 758 548
- DE-A- 3 415 147
- DE-A- 3 541 025
- FR-A- 2 233 040
- US-A- 4 025 616
- US-A- 4 241 049

## Description

The present invention relates to a dentifrice composition comprising an antibacterial agent, in particular an agent selected from the bis-biguanide group of antibacterials, which compositions are useful in the prophylaxis and/or treatment of periodontal disease and caries.

The bis-biguanide group of antibacterial agents have been disclosed in U.S. 2 684 924, 2 990 425, 2 830 006 and 2 863 019.

In particular, attention has focussed on two bis-biguanides viz chlorhexidine [N,N˝-bis(4-chlorophenyl)-3,12-diimino-2,4,11,13-tetraazatetradecanediimidamide or 1,1′-hexamethylene-bis-[5-(p-chlorophenyl)biguanide], the compound of formula (A): 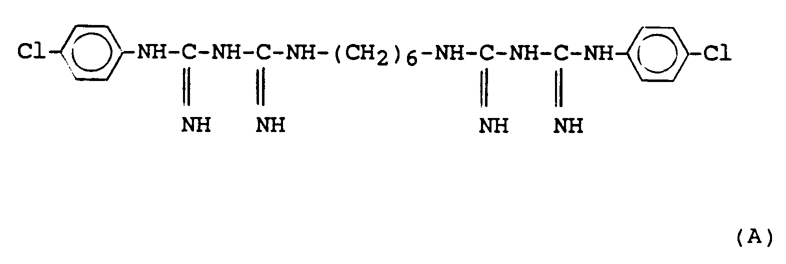
and the corresponding compound alexidene in which the chlorophenyl groups have been replaced by 2-ethylhexyl groups.

Chlorhexidine is a well established antimicrobial agent which has found use in a wide variety of applications as an antiseptic or disinfectant. In the area of oral hygiene, mouthwashes and a gel comprising chlorhexidine are commercially available. Further use of the agent has however been limited by the known propensity of chlorhexidine to form insoluble salts which remove it from solution and thereby render it unavailable. In addition, chlorhexidine has a bitter taste and tends to stain plaque brown. The latter may be minimised by using a dentifrice, rather than a mouthwash, as the abrasive incorporated therein removes plaque. The formulation of an acceptable dentifrice however provides a stern challenge, due to the multitude of components incorporated therein, each of which may be incompatible with chlorhexidine.

Various proposals have been made over the years to try and overcome the incompatibility problem, by examining various aspects of the dentifrice, for instance, the abrasive, the surfactant, the thickening agent and the process for preparation of the dentifrice.

Specific suggestions for suitable abrasives include the use of (i) abrasives coated with a cationic water soluble polymer (GB 1 506 045, to Procter + Gamble Co.), (ii) silica of a defined particle size (30% less than 5 mu) (GB 1 249 842, to Colgate-Palmolive Co.), and (iii) the use of either α-alumina monohydrate (GB 2 037 162, to Unilever NV) or α-alumina trihydrate (GB 1 302 019, to Colgate- Palmolive Co.).

Recently, EP-A-0 315 503 (to Rhone-Poulenc Chimie) (published after the earliest priority date of the present application) has disclosed the suitability of particular types of silicas, characterised by inter alia, a low anion (<1%) a content. Whilst the compatibility of these silicas with chlorhexidine and with other dentifrice ingredients was established by a spectrophotometric assay, no consideration was given to the compatibility of chlorhexidine with the other ingredients.

Specific suggestions for suitable surfactants include the use of either an amphoteric surfactant (in combination with a nonionic thickening agent such as hydroxyethyl cellulose) (JP 51 051 530, to Lion Fat and Oil KK); or a polyoxyethylene fatty ester to augment an anionic surfactant (in combination with an anionic thickening agent, such as sodium carboxymethyl cellulose) (JP 50 076 243, to Sunstar Dentifrice KK). The natural gum carrageenan was suggested to be a suitable thickening (JP 79 011 243, Lion Dentifrice Co.).

It has now been suggested that either ethanol or a water soluble alkaline earth metal salt (when the dentifrice also includes a phosphate salt) may be added, to stabilise chlorhexidine in a dentifrice comprising otherwise conventional components (US 3 989 813 and US 4 241 049, both to Colgate-Palmolive Co.). Similarly, the use of a phenol or a higher alcohol, has been proposed, to stabilise a dentifrice comprising chlorhexidine and a nonionic surfactant (JP 59 101 417, JP 59 101 418, both to Lion Corporation).

US 4 273 759 (Colgate-Palmolive Co.) discloses a chlorhexidine dentifrice comprising hydrated alumina, a nonionic surfactant and hydroxypropyl methylcellulose.

EPO 0 026 252 A1 (Blendex-Werke GmbH & Co.) discloses the use of specific zinc salts and chlorhexidine in a base comprising a nonionic thickening agent, a nonionic surfactant, and a silica abrasive. The application is directed to the problem of tooth discolouration caused by the use of chlorhexidine. No consideration is given to the compatibility of chlorhexidine with other ingredients. DE 3415147 A1 (Blendex-Werke GmbH & Co.) is similar to EPO 0 026 252 but instead uses hydrogen peroxide as an anti-discolourant. Again, a disclosure is made to a combination of the discolourant, a silica abrasive, a nonionic surfactant, and a nonionic thickening agent and chlorhexidine, but as with the other applications noted above the compatibility problems of chlorhexidine are not addressed.

GB 1 436 487 (Merck & Co.) discloses the use of alexidine dihydrofluoride in a base comprising a nonionic surfactant, a nonionic thickening agent, and insoluble sodium metaphosphate.

US 4,661,342 (to Lion Corporation) discloses an oral composition comprising a hydroxamic acid derivative for the prevention of dental caries and periodontal disease. A base containing a silica abrasive in combination with chlorhexidine, a nonionic surfactant, and a nonionic thickening agent is disclosed.

FR 2 341 302 (to Pierre Fabre SA) discloses the use of a microbiological assay for assessing the compatibility of chlorhexidine with various types of conventional dentifrice ingredient. Anionic surfactant, the abrasive bentonite and carrageenan and alginate gums were found to be unsuitable.

The appropriate sequence of addition of the various components has been identified as being of importance in the maintenance of chlorhexidine in a soluble form in the formulation (US 3 843 779 and US 3 842 168, both to Colgate-Palmolive Co.)., and JP 60 130 511, to Lion Corporation).

Several methods of overcoming the unpleasant bitter flavour associated with chlorhexidine have been disclosed, including the use, in an alumina-based dentifrice, of a predominantly peppermint flavour, moderated by the additional of an aniseed flavour (GB 2 035 084, to Unilever).

In spite of all these proposals, as far as we are aware, no dentifrice comprising an effective amount of chlorhexidine or any other bis-biguanide antibacterial agent is currently available commercially.

It has now been discovered that this problem of incompatibility can be overcome or at least mitigated by the use of a particular formulation of dentifrice.

Accordingly, the present invention provides a dentifrice comprising:
(a) a bacteriostatically effective amount of a bis-guanide compound of formula (I): 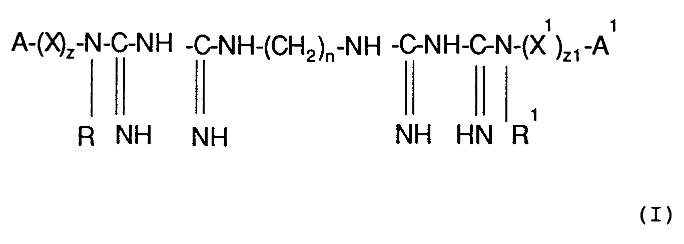 wherein
   - A and A¹: each represent
   (i) a phenyl group optionally substituted by (C₁₋₄)alkyl, (C₁₋₄) alkoxy, nitro or halogen,
   (ii) a (C₁₋₁₂) alkyl group, or
   (iii) a (C₄₋₁₂) alicyclic group;
   - X and X¹: each represent (C1-3) alkylene;
   - R and R¹: each represent hydrogen, (C₁₋₁₂) alkyl, or aryl(C₁₋₆)alkyl;
   - Z and Z¹: are each 0 or 1;
   - n: is an integer from 2 to 12;
   and the polymethylene chain (CH₂)ₙ may optionally be interrupted by oxygen or sulphur or an aromatic nucleus;
   or an orally acceptable acid addition salt thereof, in aqueous solution;
(b) a nonionic thickening agent;
(c) a nonionic surfactant; and
(d) an abrasive;
characterized in that
the abrasive is an essentially insoluble compound selected from silica having an anion content less than 1% by weight of the abrasive, zinc orthophosphate; or a mixture thereof.

The abrasive may further comprise at least one sparingly soluble salt, in combination with an agent to suppress anion formation.

Advantageously, the bis-biguanide of Formula (I) is present in the range 0.005 to 10%, preferably 0.005 to 5%, more preferably 0.005 to 2.5% by weight of the composition, calculated as the free base.

Examples of bis-biguanides of Formula (I) are chlorhexidine and alexidine, of which chlorhexidine is particularly preferred.

Suitable acid addition salts of chlorhexidine are those which have a water solubility at 20°C of at least 0.005% w/v and include digluconate, diformate, diacetate, dipropionate, dihydrochloride, dihydroiodide, dilactate, dinitrate, sulphate, and tartrate salts. Preferably the salt is the dihydrochloride, diacetate or digluconate salt.

Suitable acid addition salts of alexidine include the dihydrochloride salt.

Suitable nonionic thickening agents include (C₁₋₆)alkylcellulose ethers, for instance methylcellulose, and (C₂₋₆)alkylene oxide modified (C₁₋₆)alkylcellulose ethers, for instance hydroxypropyl methylcellulose, and mixtures thereof.

Preferably, the nonionic thickening agent is a low viscosity grade of hydroxypropyl methylcellulose which may advantageously be used in combination with a high viscosity grade of hydroxypropyl methylcellulose, the mixture being balanced to give the formulation the required viscosity.

Suitable grades of hydroxypropyl methylcellulose are marketed under the trade name 'Methocel' by Dow Chemical Corporation. The grades 'Methocel K15M' (high viscosity) and 'Methocel K100LV' are found to be particularly useful, an effective ratio thereof being in the range of from 1:50 to 1:1, preferably 1:20 to 1:2.

Advantageously, the nonionic thickening agent is present in the range 0.01 to 30%, preferably 0.1 to 15%, more preferably 1 to 5%, by weight of the dentifrice.

Suitable nonionic surfactants include, for instance polyethoxylated sorbitol monoesters (for instance, the products marketed under the trade name 'Tween' by ICI), polycondensates of ethylene oxide and propylene oxide (poloxamers) (for instance the products marketed under the trade name 'Pluronic' by BASF-Wyandotte), condensates of propylene glycol and polyethoxylated hydrogenated castor oil (for instance, cremophors).

Advantageously, the surfactant is present in the range 0.005 to 20%, preferably 0.1 to 10%, more preferably 0.1 to 5% by weight of the dentifrice.

Suitable sparingly soluble salts that may be used as an abrasive include calcium carbonate, calcium phosphates, magnesium carbonate, insoluble sodium metaphophate, and suitable mixtures thereof. The agent to suppress anion formation typically comprises a water soluble salt containing a cation which may be same as the cation of the abrasive and which forms an essentially insoluble or sparingly soluble salt with the anion of the abrasive.

Preferably the sparingly soluble salt used as an abrasive is calcium carbonate, advantageously used in combination with dicalcium phosphate, which also usefully buffers the pH of the formulation. Suitable types of calcium carbonate include both natural and synthetic chalks.

The agent to suppress anion formation may be an alkaline earth metal salt, for instance calcium chloride. The agent is preferably present in from 0.0001 to 1%, more preferably 0.005 to 0.1% by weight of the dentifrice.

The term ''essentially insoluble compound'' as used herein refers to a compound which is intrinsically insoluble in aqueous solution and includes those compounds which are listed as being ''insoluble'' in cold water in the "Handbook of Chemistry and Physics", 48th Edition, Chemical Rubber Company, Section B, Physical Constants of Inorganic Compounds. Furthermore, such compounds when used as an abrasive shall contain little if any contaminating anionic impurities. The insoluble abrasive compound contains less than 1%, more preferably less than 0.5%, of anionic impurities, based on the weight of the abrasive.

Suitable insoluble abrasives include silica, zinc orthophosphate, or mixtures thereof.

The preferred silica abrasive may be a natural amorphous silica, for instance diatomaceous earth, or a synthetic amorphous silica, for instance a precipitated silica or a silica gel, such as a silica xerogels; or mixtures thereof.

The preferred grades of synthetic amorphous silicas are those for which the manufacturing process is carefully controlled so that the level of anion impurities, particularly sulphate and silicate from sodium sulphate and sodium silicate, respectively, is kept to a minimum. Alternatively, or in addition, the level of anion impurities may be reduced to the required level by careful washing of the silica with, for instance, deionised or distilled water.

Suitable silicas include those described in EP A 0 315 503 (to Rhone-Poulenc) which are characterized as having:
(i) less than 5 X 10⁻³, preferably less than 1 X 10⁻³, more preferably less than 0.2 X 10⁻³ moles of anions per 100g of silica, and in particular less than 0.5%, preferably less than 0.1%, more preferably less than 0.02% by weight of silica of sulphate anions;
(ii) a Hammett acidity function Ho of at least 3.3, which may be determined according to the method of Walling, J. Amer. Chem. Soc., 1950, 72, 1164;
(iii) a surface in which the number of hydroxyl groups, expressed as OH/nm² is less than 15, more particularly less than 12, which number may be determined according to the method described in EP-A-0 315 503; and
(iv) a point zero charge of at least 3 and preferably between 4 and 6, which point zero charge may be determined according to the method described in EP-A-0 315 503.

Such silicas may be prepared as precipitates or gels from silicate and acid by conventional means, the crude silica product being collected and then washed with water, preferably deionised water, until the conductivity of the washings is less than 200 microsiemens cm⁻¹, preferably less than 100 microsiemens cm⁻¹, and then dried and, if necessary, ground, to give the desired sizes of particles. Alternatively, an initial washing of the crude product with water, until the washings have a conductivity of less than 2000 microsiemens cm⁻¹, may be followed by washing with an acid or an aqueous acid, for instance a mineral acid such as nitric acid, or an organic acid such as acetic acid or citric acid, until the silica has a pH of less than 8, preferably between 6 and 7.5.

Suitable silica xerogels are described in US 3 538 230.

Suitable grades of precipitated silica have BET surface areas in the range 20 to 300, preferably 20-100 m²/g and median agglomerate sizes in the range 2 to 50, preferably 5 to 30µm.

Preferred precipitated silicas are those marketed under the trade name 'Sident' by Degussa. Preferred silica xerogels are those marketed under the trade name 'Syloblanc' by W.R. Grace Corporation, Davison Chemical Division.

Suitable forms of diatomaceous earth include those marketed under the trade name 'Celite' by Johns-Manville Products Corporation, for instance 'Celite Superfine Superfloss'.

Preferably the silica abrasive comprises diatomaceous earth which may advantageously be used in combination with a synthetic amorphous silica, in particular a precipitated silica (as hereinbefore defined), which also usefully buffers the pH of the formulation and counters the otherwise off-white colour and roughness conferred upon a formulation by the use of diatomaceous earth alone. Suitably, the ratio of diatomaceous earth to synthetic amorphous silica is from 5:1 to 1:5, preferably about 1:1.

The abrasive is advantageously present in the range 1-80%, preferably 5-70%, more preferably 5-60% by weight of the dentifrice.

In another aspect of this invention it has been found that when certain of the insoluble compounds, for instance, silica, zinc orthophosphate, or plastics particles are used as the abrasive, a fluoride salt such as an alkali metal fluoride may also be incorporated into the formulation, to provide between 100 and 3000ppm, preferably 500 to 2000ppm of fluoride. Preferably the fluoride salt is an alkali metal fluoride, for instance sodium fluoride. Other fluorides that may be used include amine fluorides.

Dentifrices according to the invention may also contain a humectant, such as glycerine, sorbitol, propylene glycol or polyethylene glycol, or mixtures thereof; which humectant may be present in the range 5 to 30%, preferably 10 to 30% by weight of the dentifrice. Preferably the humectant is glycerine.

Dentifrices according to the invention may also contain other agents conventionally used in dentifrice formulations, for instance flavouring agents; colouring agents; whitening agents; preservatives and sweetening agents. It will be a appreciated that such agents (at the level employed) should be compatible with the bis-biguanide of formula (I); that is, the agent will not substantially reduce the availability of the bis-biguanide of formula (I). This may be confirmed by, for instance, determining the biological activity of the formulation, (by following the method provided in the 'Biological Data' section) in the presence and absence of the agent. In general, such agents will be a minor amount or proportion of the formulation, usually present in from 0.001 to 5% by weight of the composition.

Flavour is an important aspect of the consumer acceptability of a dentifrice. This is particularly so in the case of a dentifrice comprising a bis-biguanide of formula I, especially chlorhexidine, because of the bitter after-taste of the bis-biguanide. Surprisingly it has now been found that this can be effectively masked by an aniseed flavour.

Accordingly, in a further aspect, the present invention provides a dentifrice as hereinbefore defined which further comprises a flavouring agent having an aniseed flavour.

Flavouring agents having an aniseed flavour include anethole, which may be present in such quantity as to mask the bitter after-taste of the bis-biguanide of formula (I).

Preferably the flavour may be modified by the additional incorporation of one or more flavouring agents having a mint flavour, to balance the aniseed flavour, to give a flavour which has more general consumer acceptability but in which the aniseed flavour is still dominant.

Suitable flavouring agents having a mint flavour include peppermint, spearmint, menthol and carvone.

Preferably more than one mint flavouring agent is used, of which menthol may be the major component, accounting for between 20 and 60%, preferably between 25 and 55% by weight of the flavouring agents having a mint flavour.

Advantageously the flavour of the dentifrice may be further modified by the incorporation of flavouring agents having spicey flavours such as coriander, eugenol and eucalyptol, the aniseed flavour still being dominant.

Accordingly, in a still further aspect, the invention provides a dentifrice as hereinbefore defined comprising aniseed and mint flavours and, optionally, spicey flavours, and having a predominantly aniseed flavour.

Preferably, in such a dentifrice comprising aniseed and mint flavours, flavouring agents having an aniseed flavour comprise from 10 to 30%, more preferably 15 to 25% of the combined weights of the flavouring agents, whilst flavouring agents having a mint flavour comprise from 40 to 80, preferably 40 to 70% of the combined weight of the flavouring agents. Preferably the combined flavouring agents comprise upto 5%, more preferably upto 2% by weight of the dentifrice.

Suitable sweetening agents include saccharin and acceptable water soluble salts thereof, such as the sodium salt, and may be present in from 0.01 to 0.5%, preferably 0.05 to 0.5% by weight of the dentifrice. An auxiliary sweetener such as a thaumatin may also be included, at a level of from 0.001 to 0.1, preferably 0.005 to 0.05% by weight of the dentifrice. A suitable blend of thaumatins is marketed under the trade name 'Talin' by Tate and Lyle plc.

Accordingly, in a preferred aspect, the invention provides a dentifrice as hereinbefore defined, comprising aniseed and mint flavours, in combination with saccharin, or the sodium salt thereof, and a thaumatin.

Water, preferably deionised or distilled water, will also be present, in the range from 10 to 80%, preferably 20 to 70% by weight of the dentifrice.

The dentifrices according to the invention may have a pH within the range pH 4 to 10, preferably pH 5 to 8.

In an especially preferred aspect, the invention provides a dentifrice comprising:
(i) chlorhexidine or an acid addition salt thereof;
(ii) a (C₂₋₆)alkylene oxide modified (C₁₋₆)alkyl cellulose thickening agent;
(iii) a poloxamer surfactant; and
(iv) an abrasive which is either:
   (a) a precipitated silica in which the anion content is less than 1%, optionally in combination with diatomaceous earth, or
   (b) calcium carbonate, optionally in combination with dicalcium orthophosphate and comprising an alkaline earth salt to act as an anion suppressant agent.

The dentifrices according to the invention are prepared in a conventional manner by mixing the ingredients thereof in the required proportions and in any order which is convenient and, thereafter and if necessary, adjusting the pH. For instance, the nonionic thickening agent and the humectant and part of the water are vigourously agitated together, with heat, if necessary, to give a hydrated gel. Abrasive is then dispersed in this hydrated gel, using a heavy-duty mixing machine, with active agents, such as chlorhexidine, or a salt thereof, a fluoride salt (if present), then added, followed by nonionic surfactant and flavouring agents in the final stage; with final mixing carried out under vacuum.

It has been found that in the particular instance of a dentifrice according to the invention comprising chlorhexidine (or an acid addition salt thereof), a silica abrasive and sodium saccharin, a smoother formulation, which has improved storage stability, with respect to the formation of lumps (which are believed to be a silica-containing form of chlorhexidine saccharinate), may be obtained if chlorhexidine, or a salt thereof, is added at an earlier, rather than a later, stage of the manufacturing process.

Accordingly, in a further aspect, the invention provides a process for the preparation of a dentifrice comprising chlorhexidine, or an acid addition salt thereof, a silica abrasive, and saccharin, or a salt thereof, which process comprises the steps of:
(i) at an earlier stage of the process, substantially simultaneously adding chlorhexidine, or an acid addition salt thereof, and saccharin, or a salt thereof, and
(ii) at a later stage of the process, adding silica; and
optionally, as a preliminary step to the aforementioned steps, forming a hydrated gel by the admixing of water, humectant and nonionic thickening agent.

The following examples illustrate the invention.

### Example 1

### Silica Based Toothpaste

| | |
|---|---|
| Chlorhexidine digluconate | 1.00% |
| Glycerin | 18.00 |
| Hydroxypropyl Methylcellulose | 3.60 |
| Silica¹ | 16.00 |
| Sodium fluoride | 0.23 |
| Flavour | 1.00 |
| Poloxamer 338 | 2.00 |
| Deionized Water | q.s. |

| | |
|---|---|
| ¹ The silica was prepared by washing Syloblanc 31 (available from W.R. Grace Corporation) with deionised water (twice), followed by drying at 50°C. | |

### Example 2

### Diatomaceous Earth Based Toothpaste

| | |
|---|---|
| Chlorhexidine digluconate | 1.00% |
| Glycerin | 18.00 |
| Hydroxypropyl Methylcellulose | 3.60 |
| Diatomaceous earth | 16.00 |
| Sodium Fluoride | 0.23 |
| Flavour | 1.00 |
| Poloxamer 338 | 2.00 |
| Deionized Water | q.s. |

### Example 3

### Zinc Orthphosphate containing Toothpaste

| | |
|---|---|
| Chlorhexidine digluconate | 1.00% |
| Glycerin | 24.00 |
| Hydroxypropyl Methylcellulose | 5.00 |
| Zinc orthophosphate | 16.00 |
| Sodium Fluoride | 0.23 |
| Flavour | 1.00 |
| Poloxamer 338 | 2.00 |
| Deionized Water | q.s. |

### Example 4

### Diatomaceous Earth/Silica Based Toothpaste

| | |
|---|---|
| Chlorhexidine digluconate | 1.00% |
| Glycerin | 18.00 |
| Hydroxypropyl Methylcellulose¹ | 3.60 |
| Diatomaceous earth | 16.00 |
| Silica² | 8.00 |
| Sodium Fluoride | 0.23 |
| Flavour³ | 1.00 |
| Sodium saccharinate | 0.10 |
| Thaumatin | 0.02 |
| Poloxamer 338 | 2.00 |
| Deionized Water | q.s. |

| | |
|---|---|
| ¹ a blend of 'Methocel K15M' and 'Methocel K100LV' in the ratio 1:5 | |
| ² 'Sident 9' available from Degussa | |
| ³ see Example 5 | |

### Example 5

### Flavour for use in the dentifrice of Example 4

| | |
|---|---|
| Peppermint oil | 16.0% |
| Anethole | 24.0 |
| Menthol | 44.9 |
| L-Carvone | 10.7 |
| Methyl acetate | 1.6 |
| Eucalyptol | 5.3 |
| Limonene | 0.5 |
| Eugenol | 0.5 |
| Coriander Oil | 2.0 |

The flavour composition masked the bitter after-taste of chlorhexidine in the dentifrice and conferred a predominantly aniseed flavour to the dentifrice.

### Example 6

### Process for the preparation of the dentifrice of Example 4

The dentifrice of Example 4 may be prepared by the following process:
1. Hydroxypropyl methylcellulose was slurried in glycerin (10% of the total volume).
2. Poloxamer 338 was dissolved in deionised water to give a 20% solution.
3. Sodium saccharin was dissolved in deionised water to give a 33% solution.
4. Thaumatin was dissolved in deionised water to give a 5% solution.
5. Deionized water (30% of total volume) was added to a heavy duty mixing vessel, followed by the hydroxypropyl methylcellulose slurry and the saccharin solution and this was then mixed for 5 minutes under vacuum.
6. Chlorhexidine gluconate solution was added and the mixture blended for a further 10 minutes under vacuum.
7. The remainder of the deionized water and glycerin were added, followed by mixing for a further 10 minutes under vacuum.
8. Silica and diatomaceous earth were sucked into the mixing vessel over 20 minutes with mixing, the mixture being mixed for a further 10 minutes, with vacuum being retained throughout.
9. The flavours and the thaumatin solution were then added, followed by mixing for a further 10 minutes under vacuum.
10. The poloxamer 338 solution was added, followed by mixing for a further 10 minutes under vacuum.
11. Sodium fluoride was added, followed by mixing for a further 15 minutes under vacuum.

### Biological Data

The data in the following table represents the potency of chlorhexidine in the dentifrice formulation, compared with a control containing chlorhexidine digluconate alone in aqueous solution and in the absence of the other agents listed. The assay is a standard agar diffusion method, using M. luteus as the assay organism.

| | Potency % |
|---|---|
| Example 1 | 59 |
| 2 | 90 |
| 3 | 90 |
| Control | 100 |

The data show that when chlorhexidine is incorporated into a dentifrice formulation according to the present invention, chlorhexidine substantially retains its potency, thereby allowing it to exert its intrinsic bacteriostatic activity within the oral cavity.

## Claims

1. A dentifrice comprising:
(a) a bacteriostatically effective amount of a bis-guanide compound of formula (I): 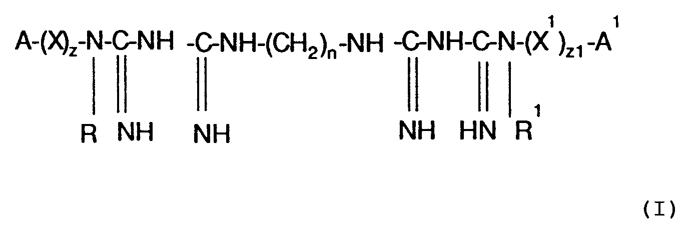 wherein
A and A¹ each represent
(i) a phenyl group optionally substituted by (C₁₋₄)alkyl, (C₁₋₄) alkoxy, nitro or halogen,
(ii) a (C₁₋₁₂) alkyl group, or
(iii) a (C₄₋₁₂) alicyclic group;
X and X¹ each represent (C1-3) alkylene;
R and R¹ each represent hydrogen, (C₁₋₁₂) alkyl, or aryl(C₁₋₆)alkyl;
Z and Z¹ are each 0 or 1;
n is an integer from 2 to 12;
and the polymethylene chain (CH₂)ₙ may optionally be interrupted by oxygen or sulphur or an aromatic nucleus;
or an orally acceptable acid addition salt thereof, in aqueous solution;
(b) a nonionic thickening agent;
(c) a nonionic surfactant; and
(d) an abrasive ;
characterized in that
the abrasive is an essentially insoluble compound selected from silica or zinc orthophosphate; or a mixture thereof having an anion content less than 1% by weight of the abrasive.

2. A dentrifice as claimed in claim 1 in which the bis-biguanide of formula (I) is chlorhexidine or alexidine, or an orally acceptable acid additional salt thereof.

3. A dentrifice as claimed in claim 1 or claim 2 in which the nonionic thickening agent is a (C₁₋₆) alkyl cellulose ether, or a (C₂₋₆) alkylene-oxide modified (C₁₋₆) alkylcellulose ether, or a mixture thereof.

4. A dentifrice as claimed in any of claims 1 to 3 in which the abrasive comprises a silica abrasive having an anion content less than 1% by weight of the abrasive which is a natural amorphous silica, or a synthetic amorphous silica, or a mixture thereof.

5. A dentifrice as claimed in claim 4 in which the natural amorphous silica comprises diatomaceous earth.

6. A dentifrice as claimed in claim 4 in which the abrasive comprises diatomaceous earth and precipitated silica.

7. A dentifrice as claimed in claim 4 in which the ratio of diatomaceous earth to precipitated silica is from 5:1 to 1:5.

8. A dentifrice as claimed in any one of claims 4 to 7 in which the anion impurity is sulphate anions.

9. A dentifrice as claimed in any one of claims 4 to 8 comprises flavouring agents having an aniseed and, optionally, a mint and a spicey flavour, the dentifrice having a predominantly aniseed flavour.

10. A dentifrice as claimed in any one of claims 4 to 9 which further comprises a silica abrasive having an anion content less than 1% by weight of the abrasive in combination with at least one sparingly soluble salt , and an agent to suppress anion formation with at least one sparingly soluble salt and an agent to suppress anion formation.

11. A dentifrice as claimed in claim 10 wherein the sparingly soluble salt is selected from calcium carbonate, calcium phosphate, dicalcium orthophosphate, magnesium carbonate, or insoluble sodium metaphosphate.

12. A process for the preparation of a dentifrice comprising chlorhexidine, or an acid addition salt thereof, silica and saccharin, or a salt thereof, which process comprises the steps of:
(i) at an earlier stage of the process, substantially simultaneously adding chlorhexidine, or an acid addition salt thereof;
(ii) at a later stage of the process, adding silica in which the anion content is less than 1%;
with optionally, as a preliminary to the aforementioned steps, forming a hydrated gel by the admixing of water, humectant and nonionic thickening agent.

13. A dentifrice comprising:
(i) chlorhexidine or an acid addition salt thereof;
(ii) a (C₂₋₆) alkylene oxide modified (C₁₋₆) alkyl cellulose thickening agent;
(iii) a poloxamer surfactant; and
(iv) an abrasive which is a precipitated silica in which the anion content is less than 1%, optionally in combination with diatomaceous earth.

14. A dentifrice as defined in claim 1 for use in oral hygiene.

## Patentansprüche

1. Zahnpflegemittel, umfassend
(a) eine bakteriostatisch wirksame Menge einer Bis-guanidverbindung der allgemeinen Formel (I): 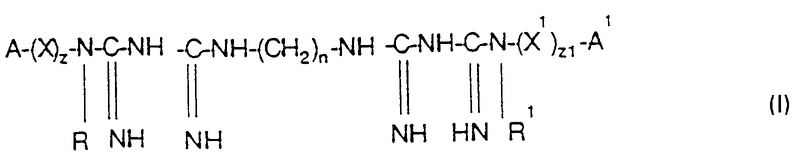 in der jeder Rest A und A¹
(i) ein Phenylrest, der gegebenenfalls mit C₁₋₄-Alkyl-, C₁₋₄-Alkoxy-, Nitro- oder Halogengruppen substituiert ist, oder
(ii) ein C₁₋₁₂-Alkylrest, oder
(iii) ein C₄₋₁₂-alicyclischer Rest ist,
jeder Rest X und X¹ ein C₁₋₃-Alkylenrest ist,
jeder Rest R und R¹ ein Wasserstoffatom, ein C₁₋₁₂-Alkylrest oder ein Aryl-C₁₋₆-Alkylrest ist,
z und z¹ den Wert 0 oder 1 haben,
n eine ganze Zahl von 2 bis 12 ist,
und die Polymethylenkette (CH₂)ₙ gegebenenfalls mit einem Sauerstoff-oder Schwefelatom oder einem aromatischen Ring unterbrochen sein kann,
oder ein im Mund verträgliches Säureadditionssalz davon, in wäßriger Lösung,
(b) ein nicht-ionisches Verdickungsmittel,
(c) ein nicht-ionisches Tensid, und
(d) ein Schleifmittel, dadurch gekennzeichnet, daß das Schleifmittel eine im wesentlichen unlösliche Verbindung ausgewählt aus Siliciumdioxid oder Zinkorthophosphat oder deren Gemisch ist, mit einem Anion-Gehalt von weniger als 1 Gew.-% des Schleifmittels.

2. Zahnpflegemittel nach Anspruch 1, wobei das Bis-guanid der allgemeinen Formel (I) Chlorhexidin oder Alexidin oder ein im Mund verträgliches Säureadditionssalz davon ist.

3. Zahnpflegemittel nach Anspruch 1 oder Anspruch 2, wobei das nicht-ionische Verdickungsmittel ein C₁₋₆-Alkylcelluloseether oder ein C₂₋₆-Alkylenoxid-modifizierter C₁₋₆-Alkylcelluloseether oder deren Gemisch ist.

4. Zahnpflegemittel nach einem der Ansprüche 1 bis 3, wobei das Schleifmittel ein Siliciumdioxidschleifmittel mit einem Anion-Gehalt von weniger als 1 Gew.-% des Schleifmittels umfaßt, das ein natürliches oder synthetisches, amorphes Siliciumdioxid oder deren Gemisch ist.

5. Zahnpflegemittel nach Anspruch 4, wobei das natürliche amorphe Siliciumdioxid Kieselgur umfaßt.

6. Zahnpflegemittel nach Anspruch 4, wobei das Schleifmittel Kieselgur und gefällte Kieselsäure umfaßt.

7. Zahnpflegemittel nach Anspruch 4, in dem das Verhältnis von Kieselgur zu gefällter Kieselsäure bei 5:1 bis 1:5 beträgt.

8. Zahnpflegemittel nach einem der Ansprüche 4 bis 7, in dem der Anion-Fremdbestandteil aus Sulfatanionen besteht.

9. Zahnpflegemittel nach einem der Ansprüche 4 bis 8, umfassend Geschmacksstoffe mit einem Anis- und gegebenenfalls einem Mint- und einem würzigen Geschmack. wobei das Zahnpflegemittel überwiegend Anis-Geschmack hat.

10. Zahnpflegemittel nach einem der Ansprüche 4 bis 9, umfassend außerdem ein Siliciumdioxid-Schleifmittel mit einem einem Anion-Gehalt von weniger als 1 Gew.-% des Schleifmittels in Verbindung mit mindestens einem schwerlöslichen Salz, und ein Mittel zur Unterdrückung der Anionbildung mit mindestens einem schwerlöslichen Salz und ein Mittel zur Unterdrückung der Anionbildung.

11. Zahnpflegemittel nach Anspruch 10, wobei das schwerlösliche Salz ausgewählt ist aus Calciumcarbonat, Calciumphosphat, Dicalciumorthophosphat, Magnesiumcarbonat oder unlöslichem Natriummetaphosphat.

12. Verfahren zur Herstellung eines Zahnpflegemittels umfassend Chlorhexidin oder dessen Säureadditionssalz, Siliciumdioxid und Saccharin, oder dessen Salz, wobei das Verfahren die folgenden Schritte umfaßt:
(i) in einem früheren Stadium des Verfahrens im wesentlichen gleichzeitige Zugabe von Chlorhexidin oder dessen Säureadditionssalz,
(ii) in einem späteren Stadium des Verfahrens Zugabe von Siliciumdioxid mit einem Anion-Gehalt von weniger als 1 Gew.-%,
mit gegebenenfalls einer den zuvorgenannten Schritten vorhergehenden Bildung eines wasserhaltigen Gels durch Zumischen von Wasser, Anfeuchtemittel und nicht-ionischem Verdickungsmittel.

13. Zahnpflegemittel, umfassend
(i) Chlorhexidin oder dessen Säureadditionssalz,
(ii) ein C₂₋₆-Alkylenoxid-modifiziertes C₁₋₆-Alkylcellulose-Verdickungsmittel,
(iii) ein Poloxamer-Tensid, und
(iv) ein Schleifmittel, das gefällte Kieselsäure mit einem Anion-Gehalt von weniger als 1 Gew.-%ist, gegebenenfalls in Verbindung mit Kieselgur.

14. Zahnpflegemittel nach Anspruch 1 zur Verwendung bei der Mundhygiene.

## Revendications

1. Dentifrice comprenant :
(a) une quantité efficace du point de vue bactériostatique d'un composé bis-guanide représenté par la formule (I) : 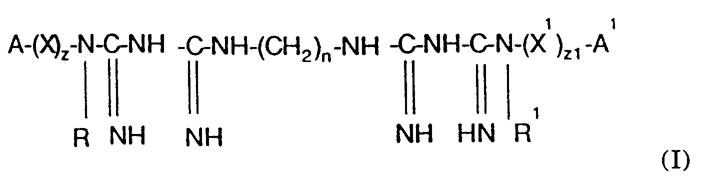 dans laquelle :
A et A¹ représentent chacun
(i) un groupe phényle éventuellement substitué par un groupe alkyle en C₁₋₄, alcoxy en C₁₋₄, nitro ou un atome d'halogène,
(ii) un groupe alkyle en C₁₋₁₂, ou
(iii) un groupe alicyclique en C₄₋₁₂;
X et X¹ représentent chacun un groupe alkylène en C₁₋₃;
R et R¹ représentent chacun un atome d'hydrogène, un groupe alkyle en C₁₋₁₂ ou aryl-alkyle en C₁₋₆;
z et z¹ sont chacun 0 ou 1;
n est un entier de 2 à 12;
et la chaîne polyméthylène (CH₂)ₙ peut être éventuellement interrompue par un atome d'oxygène ou de soufre, ou par un noyau aromatique;
ou d'un sel d'addition d'acide de celui-ci acceptable par voie orale, en solution aqueuse;
(b) un agent épaississant non ionique;
(c) un tensioactif non ionique; et
(d) un abrasif;
caractérisé en ce que l'abrasif est un composé essentiellement insoluble choisi parmi la silice ou l'orthophosphate de zinc, ou un mélange de ceux-ci ayant une teneur en anions inférieure à 1% en poids de l'abrasif.

2. Dentifrice suivant la revendication 1, dans lequel le composé bis-guanide de formule (I) est la chlorhexidine ou l'alexidine, ou un sel d'addition d'acide de celles-ci acceptables par voie orale.

3. Dentifrice suivant les revendications 1 ou 2, dans lequel l'agent épaississant non ionique est un éther cellulosique d'alkyle en C₁₋₆ ou un éther cellulosique d'alkyle en C₁₋₆ modifié par un oxyde d'alkylène en C₂₋₆ ou un mélange de ceux-ci.

4. Dentifrice suivant l'une quelconque des revendications 1 à 3, dans lequel l'abrasif comprend un abrasif de type silice ayant une teneur en anions inférieure à 1% en poids de l'abrasif qui est une silice amorphe naturelle, ou une silice amorphe synthétique ou un mélange de celles-ci.

5. Dentifrice suivant la revendication 4, dans lequel la silice amorphe naturelle comprend une terre d'infusoires.

6. Dentifrice suivant la revendication 4, dans lequel l'abrasif comprend une terre d'infusoires et une silice précipitée.

7. Dentifrice suivant la revendication 4, dans lequel le rapport de la terre d'infusoires à la silice précipitée est compris entre 5:1 et 1:5.

8. Dentifrice suivant l'une quelconque des revendications 4 à 7, dans lequel l'impureté anionique est constituée par des anions sulfate.

9. Dentifrice suivant l'une quelconque des revendications 4 à 8, qui comprend des arômes ayant un parfum à l'anis et, éventuellement une menthe et un parfum d'épice, le dentifrice ayant un parfum à l'anis prédominant.

10. Dentifrice suivant l'une quelconque des revendications 4 à 9, qui comprend de plus un abrasif de type silice ayant une teneur en anions inférieure à 1% en poids de l'abrasif en association avec au moins un sel difficilement soluble et un agent pour supprimer la formation d'anions.

11. Dentifrice suivant la revendication 10, dans lequel le sel difficilement soluble est choisi par le carbonate de calcium, le phosphate de calcium, l'orthophosphate dicalcique, le carbonate de magnésium ou le métaphosphate de sodium insoluble.

12. Procédé pour préparer un dentifrice comprenant de la chlorhexidine ou un sel d'addition d'acide de celle-ci, de la silice et de la saccharine ou un sel de celle-ci, qui comprend les étapes suivantes :
(i) dans une des premières phases du traitement, l'addition pratiquement simultanée de chlorhexidine ou d'un sel d'addition d'acide de celle-ci, et de saccharine ou d'un sel de celle-ci;
(ii) dans une phase ultérieure du traitement, l'addition d'une silice dans laquelle la teneur en anions est inférieure à 1%;
avec éventuellement, en tant que phase précédant celles-ci, la formation d'un gel hydraté par mélange d'eau, d'humectant et d'un agent épaississant non ionique.

13. Dentifrice comprenant :
(i) de la chlorhexidine ou un sel d'addition d'acide de celle-ci;
(ii) un agent épaississant constitué par un éther cellulosique d'alkyle en C₁₋₆ modifié par un oxyde d'alkylène en C₂₋₆;
(iii) un tensioactif de type poloxamère; et
(iv) un abrasif qui est une silice précipitée dans laquelle la teneur en anions est inférieure à 1%, éventuellement en association avec une terre d'infusoires.

14. Dentifrice suivant la revendication 1, utile dans l'hygiène orale.
